# EUROPEAN PATENT SPECIFICATION

(11) **EP 2 882 443 B1**
(45) Date of publication and mention of the grant of the patent: **16.01.2019**
(21) Application number: 13828399.9
(22) Date of filing: 09.08.2013
(51) Int. Cl.: A61K 31/216, A61K 31/192, A61K 36/53, A61K 36/534, A61K 31/56, A61K 31/7032, A61K 31/7034, A61K 36/73, A61P 25/28, A61P 25/16

(54) **PLANT EXTRACTS FOR IMPROVING COGNITIVE HEALTH AND FUNCTION**
PFLANZENEXTRAKTE ZUR VERBESSERUNG DER KOGNITIVEN GESUNDHEIT UND KOGNITIVER FUNKTIONEN
EXTRAITS DE PLANTE POUR AMÉLIORER LA SANTÉ ET LA FONCTION COGNITIVES

(30) Priority: 09.08.2012 US 201261681414 P
(43) Date of publication of application: 17.06.2015
(73) Proprietor: Kemin Industries, Inc., Des Moines, Iowa 50317 (US)
(72) Inventor: CEDDIA, Michael, Ankeny, Iowa 50023 (US); HERRLINGER, Kelli, Johnston, Iowa 50131 (US); LEWIS, Brandon, Grimes, Iowa 50111 (US); FENG, Harry, West Des Moines, Iowa 50266 (US)
(74) Representative: Evans, Jacqueline Gail Victoria
(86) International application number: PCT/US2013/054274
(87) International publication number: WO 2014/026081

(56) References cited:
- EP-A1- 1 930 019
- WO-A1-2009/056208
- WO-A1-2009/056208
- WO-A2-2008/090474
- JP-A- 2006 199 666
- JP-A- 2011 256 118
- US-A- 6 140 363
- US-A1- 2007 208 029
- US-A1- 2012 076 878
- US-A1- 2012 149 766
- US-B2- 7 972 633
- ALKAM ET AL: "A natural scavenger of peroxynitrites, rosmarinic acid, protects against impairment of memory induced by Abeta25-35", BEHAVIOURAL BRAIN RESEARCH, ELSEVIER, AMSTERDAM, NL, vol. 180, no. 2, 11 May 2007 (2007-05-11), pages 139-145, XP022070544, ISSN: 0166-4328, DOI: 10.1016/J.BBR.2007.03.001
- PARK D H ET AL: "Subchronic administration of rosmarinic acid, a natural prolyl oligopeptidase inhibitor, enhances cognitive performances", FITOTERAPIA, IDB HOLDING, MILAN, IT, vol. 81, no. 6, 1 September 2010 (2010-09-01), pages 644-648, XP027172583, ISSN: 0367-326X [retrieved on 2010-03-15]
- DATABASE WPI Week 201068 Thomson Scientific, London, GB; AN 2010-M36674 XP002751487, & CN 101 773 488 A (UNIV QINGDAO) 14 July 2010 (2010-07-14)
- TISSERAT BRENT ET AL: "Spearmint plantlet culture system as a means to study secondary metabolism.", METHODS IN MOLECULAR BIOLOGY (CLIFTON, N.J.) 2009, vol. 547, 2009, pages 313-324, XP008178219, ISSN: 1064-3745
- FARR S A ET AL: "Antioxidant extracts from rosemary and spearmint improve learning, memory and reduce oxidative stress in samp8 mice", ABSTRACTS OF THE ANNUAL MEETING OF THE SOCIETY FOR NEUROSCIENCE, SOCIETY FOR NEUROSCIENCE, WASHINGTON, DC, US , vol. 43 9 November 2013 (2013-11-09), pages 1-2, XP008178172, ISSN: 0190-5295 Retrieved from the Internet: URL:http://www.abstractsonline.com/plan/Ab stractPrintView.aspx?mID=3236&sKey=c2f065a 0-271d-44dc-9008-2ac011a36180&cKey=a46cde5 a-31d7-43bc-9da4-ab073b19bb91 [retrieved on 2015-11-20]
- FONSECA BRENDA (CORRESPONDENCE) ET AL: "Effects of a distinct phenolic complex on working memory performance in healthy men and women with age - associated memory impairment", NEUROLOGY, LIPPINCOTT WILLIAMS & WILKINS, PHILADELPHIA, US , vol. 84, no. 14 Supplement P7.105 6 April 2015 (2015-04-06), pages 1-2, XP008178171, ISSN: 0028-3878 Retrieved from the Internet: URL:http://www.neurology.org/content/84/14 _Supplement/P7.105 [retrieved on 2015-04-08]
- PENGELLY, A ET AL.: 'Short-term study on the effects of rosemary on cognitive function in an elderly population.' JOURNAL OF MEDICINAL FOOD vol. 15, no. 1, 04 January 2012, pages 10 - 17, XP055197788
- FARR SUSAN A ET AL: "The antioxidants alpha-lipoic acid and N-acetylcysteine reverse memory impairment and brain oxidative stress in aged SAMP8 mice", JOURNAL OF NEUROCHEMI, WILEY INTERSCIENCE, NEW YORK, NY, US, vol. 84, no. 5, 1 March 2003 (2003-03-01), pages 1173-1183, XP007914324, ISSN: 0022-3042, DOI: 10.1046/J.0022-3042.2003.01580.X
- Jennifer Prince: "Spearmint Extract Neumentix May Outperform Standard Rosmarinic Acid as Anti-Inflammatory", Nutritional Outlook, 13 June 2017 (2017-06-13), pages 1-3, XP055481471, Retrieved from the Internet: URL:http://www.nutritionaloutlook.com/herb s-botanicals/spearmint-extract-neumentix-m ay-outperform-standard-rosmarinic-acid-ant i-inflammatory [retrieved on 2018-06-06]
- Neumentix Product Specifications
- CIRLINI MARTINA ET AL: "Phenolic and Volatile Composition of a Dry Spearmint (Mentha spicata L.) Extract.", MOLECULES (BASEL, SWITZERLAND) 03 AUG 2016, vol. 21, no. 8, 3 August 2016 (2016-08-03) , ISSN: 1420-3049
- SARTORI ANDREA C ET AL: "The impact of inflammation on cognitive function in older adults: implications for healthcare practice and research.", THE JOURNAL OF NEUROSCIENCE NURSING : JOURNAL OF THE AMERICAN ASSOCIATION OF NEUROSCIENCE NURSES AUG 2012, vol. 44, no. 4, August 2012 (2012-08), pages 206-217, ISSN: 1945-2810

## Description

### Background of the Invention

The invention relates generally to plant extracts that enhance, improve or sustain cognitive health and function and, more specifically, to the administration of a water extract of a spearmint plant comprising rosmarinic acid to improve learning, memory, attention, alertness, executive function, verbal fluency, processing speed, and/or cognitive flexibility and associated behaviors.

There is a strong demand for products that can improve cognitive health or function and the market for these products has continued to grow in recent years despite the unfavorable economic pressures. Some of this growth can be attributed to growth of aging population, which is especially true in Asia and the US. Worldwide cognitive health ingredient sales are around $455 million. Frost and Sullivan has predicted an annual growth rate in this area to 12% from 2016 to 2019.

Major ingredients for cognitive health currently include phosphatidylserine (PS), CoQ10, omega-3(marine oils/algae oils), citicoline, ginko and ginseng. Of the largest cognitive health ingredients, phosphatidylserine is the only one with a FDA approved qualified claim. With increasing scientific evidence to support the claim, the ingredient has be enjoying double digit growth in sales. In 2010, DHA and EPA health claims for brain function, heart health and vision obtained a positive opinion from EFSA in Europe. Citicoline is promoted as an ingredient that prevents neuronal degeneration and improves memory.
Carnosic acid (CA) is one of the most abundant polyphenolic compounds present in *Rosmarinus officinalis* (rosemary) and *Salvia officinalis* (sage). Currently, CA is already available on the market as an ingredient or as a finished dietary supplement. Purity of these products varies between 25% and 60%, with one HPLC grade product listed as 98%. In addition, there are rosemary extracts that are standardized to CA available in powder form (standardized to 5-30% CA) or as a dietary supplement (300 mg capsule standardized to 6% CA with a recommended dose of three capsules per day). The percentage of CA in these rosemary extracts varies from 5-60%. Rosemary has a combination of phenolic diterpenes (carnosic acid and carnosol), phenolic acids (caffeic acid and rosmarinic acid), and flavonoids. A diterpene is a combination of 4 isoprene units and is known to be antimicrobial and anti-inflammatory (Bisio et al. Antimicrobial activity of the ornamental species Salvia corrugata, a potential new crop for extractive purposes. J Agric Food Chem 56: 10468-10472, 2008; Sato et al. Antibacterial novel phenolic diterpenes from Podocarpus macrophyllus D. Don. Chem Pharm Bull (Tokyo) 56: 1691-1697, 2008; Su et al. Anti-inflammatory activities of furanoditerpenoids and other constituents from Fibraurea tinctoria. Bioorg Med Chem 16: 9603-9609, 2008; Wang et al. Triptolide inhibits the differentiation of Th17 cells and suppresses collagen-induced arthritis. Scand J Immunol 68: 383-390, 2008). These isoprene units can be linked together as a linear chain or arranged into a ring formation. CA is considered the main active ingredient in *Rosmarinus officinalis,* along with its derivative, carnosol.

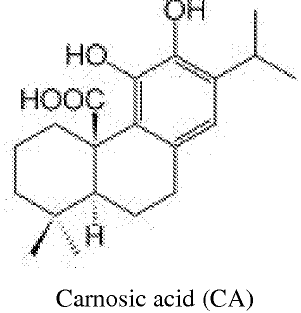

A large portion of the *in vitro* studies available evaluated CA for antioxidant protection. In fact, the antioxidant activity of rosemary has been primarily attributed to CA (Aruoma et al. An evaluation of the antioxidant and antiviral action of extracts of rosemary and provençal herbs. Food and Chemical Toxicology 34: 449-456, 1996; Pérez-Fons et al. Rosemary (Rosmarinus officinalis) diterpenes affect lipid polymorphism and fluidity in phospholipid membranes. Archives of Biochemistry and Biophysics 453: 224-236, 2006; Wang et al. Augmentation by Carnosic Acid of Apoptosis in Human Leukaemia Cells Induced by Arsenic Trioxide via Upregulation of the Tumour Suppressor PTEN. The Journal of International Medical Research 36: 682-690, 2008). Neuro-protective effects of CA were assessed using the glutamate model of oxidative stress. The study demonstrated that CA protected neurons both in vivo and in vitro against severe glutamate insult (Sato et al. Carnosic acid, a electrophilic compound, protects neurons both in vitro and in vivo through activation of the Keap1/Nrf2 pathway via alkylation of targeted cysteines on Keap1. Journal of Neurochemistry 104: 1116-1131, 2008). The more recent literature on CA discussed it potential anti-inflammatory properties. In addition to the work which showed that CA had anti-inflammatory activity due to down-regulation of NF-κB transcription factors, other studies have evaluated additional mechanisms for the anti-inflammatory properties of CA (Laughton et al. Inhibition of mammalian 5-lipoxygenase and cyclo-oxygenase by flavonoids and phenolic dietary additives: Relationship to antioxidant activity and to iron ion-reducing ability. Biochemical Pharmacology 42: 1673-1681, 1991; Poeckel et al. Carnosic acid and carnosol potently inhibit human 5-lipoxygenase and suppress pro-inflammatory responses of stimulated human polymorphonuclear leukocytes. Biochem Pharmacol 76: 91-97, 2008; Rau et al. Carnosic Acid and Carnosol, Phenolic Diterpene Compounds of the Labiate Herbs Rosemary and Sage, are Activators of the Human Peroxisome Proliferator-Activated Receptor Gamma. Planta Med 72: 881-887, 2006).

The *in vitro* data above is supported by *in vivo* data on the antioxidant capabilities of CA. Recent *in vivo* literature indicates anti-obesity and anti-glycative effects of CA (Alarcon-Aguilar et al. Investigation on the hypoglycaemic effects of extracts of four Mexican medicinal plants in normal and alloxan-diabetic mice. Phytother Res 16: 383-386, 2002; Hsieh et al. Low-density lipoprotein, collagen, and thrombin models reveal that Rosemarinus officinalis L. exhibits potent antiglycative effects. J Agric Food Chem 55: 2884-2891, 2007; Ninomiya et al. Carnosic acid, a new class of lipid absorption inhibitor from sage. Bioorganic & Medicinal Chemistry Letters 14: 1943-1946, 2004; Takahashi et al. Carnosic acid and carnosol inhibit adipocyte differentiation in mouse 3T3-L1 cells through induction of phase 2 enzymes and activation of glutathione metabolism. Biochem Biophys Res Commun 382: 549-554, 2009).

Rosmarinic acid (RA) is one of the major components found in spearmint and is an important contributor to its antioxidant capacity (Fletcher et al. Heat stress reduces the accumulation of rosmarinic acid and the total antioxidant capacity in spearmint (Mentha spicata L). Journal of the Science of Food and Agriculture 85: 2429-2436, 2005). RA, a naturally occurring phenolic compound, is an ester of caffeic acid and 3,4-dihydroxyphenyllactic acid. Its structure consists of a carbonyl group, unsaturated double bond, and carboxylic acid between two phenolic rings. RA has shown several biological activities, such as anti-inflammatory, anti-mutagenic, antibacterial, antidepressant, HIV-1 inhibitory, antioxidant, and antiviral properties. These properties have made RA an attractive ingredient for the pharmaceutical and cosmetic industries. RA has been used topically in Europe as a non-steroidal anti-inflammatory drug (Ritschel et al. Percutaneous absorption of rosmarinic acid in the rat. Methods and Findings in Experimental and Clinical Pharmacology 11: 345-352, 1989). Due to its extensive use as a flavoring agent and preservative in the food industry, RA is regarded as a daily-consumed safe ingredient (Alkam et al. A natural scavenger of peroxynitrites, rosmarinic acid, protects against impairment of memory induced by Aβ25-35. Behavioural Brain Research 180: 139-145, 2007).

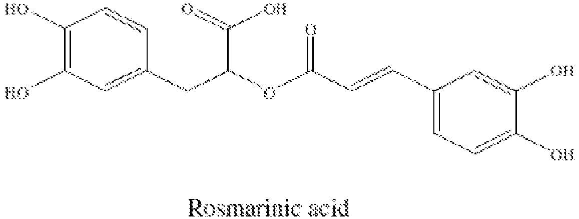

Evidence of RA's non-specific protective properties has been found within the brain. Improved anti-oxidant activity of the brain was demonstrated following RA administration to aging mice which resulted in increased activities of superoxide dismutase (SOD) and catalase (CAT) in the brain, while decreasing malondialdehyde (MDA) (Shou et al. Rosmarinic acid attenuates D-galactose induced behavior impairment in mice and its mechanism. 2010, p. 1723-1726). These data demonstrate the non-specific protective properties of RA as an antioxidant; however, no previous data has demonstrated RA's ability to affect the brain in specific regions or on specific clinical outcomes.

In vivo, three studies have evaluated administration of RA. These studies have administered RA either orally or IP in intracranial injury models or a stress model that were used to represent specific cognitive disease states (Alkam et al. A natural scavenger of peroxynitrites, rosmarinic acid, protects against impairment of memory induced by Aβ25-35. Behavioural Brain Research 180: 139-145, 2007; Park et al. Subchronic administration of rosmarinic acid, a natural prolyl oligopeptidase inhibitor, enhances cognitive performances. Fitoterapia 81:644-648, 2010; Zhou et al. Rosmarinic acid attenuates D-galactose induced behavior impairment in mice and its mechanism. Intl Conf BMEI 4:1723-1726, 2010). Although RA showed benefit in these models, they are not a validated model for evaluation of normal aging cognitive changes (as in SAMP8). In addition, it is unknown if the mechanisms of action are specific or non-specific due to antioxidant effects. Currently there are no published human studies evaluating RA supplementation alone or through use of a spearmint extract.

Learning and memory can be divided into two main categories, declarative and procedural. Declarative has temporal, spatial and associative memory components. This relates to learning and memory that has a conscious component requiring attention and alertness. In humans this relates to the acquisition, recognition and memory of discrete events, places, people, and facts. Declarative learning and memory are measured in the currently described animal study through T-maze acquisition, T-maze retention, and object recognition. Procedural learning and memory can be formed when a declarative memory task becomes routine or habitual and was measured in the current animal study through the lever press. This relates to learning and memory that does not have a conscious component, which in humans is a habit or skill, such a riding a bike. Declarative tasks are thought of as hippocampal initiated, while procedural tasks are primarily linked to the caudate regions of the brain.

For evaluation of cognitive effects resulting from aging, the senescence accelerated mouse-prone 8 (SAMP8) is proven model of accelerated aging that develops deficits in learning and memory by 8 months of age (Yagi H, Katoh S, Akiguchi I, Takeda T (1988) Age-related deterioration of ability of acquisition in memory and learning in senescence accelerated mouse: SAM-P/8 as an animal model of disturbances in recent memory. Neurosci Biobehav Rev. 474, 86-93; Flood JF, Morley JE (1998) Learning and memory in the SAMP8 mouse. Neurosci Biobehav Rev. 22, 1-20). The SAMP8 mouse has natural mutations leading to age-related increases in the amyloid precursor protein (AβPP) and amyloid beta (Aβ) in the brain and increased free radical production in the central nervous system resulting in learning and memory deficits (Butterfield DA, Howard BJ, Yatin S, Akkeb KL, Carney JM (1997) Free radical oxidation of brain proteins in accelerated senescence and its modulation by N-tert-butyl-α-phenylnitrone. Prac Natl Acad Sci USA. 94, 674-678; Sato E, Kurokawa T, Oda N, Ishibashi S (1996) Early appearance of abnormality of microperoxisomal enzymes in the cerebral cortex of senescence-accelerated mouse. Mech Ageing Dev. 92, 175-184). The SAMP8 model has been used in the literature for the evaluation of the cognitive benefits of various nutritional ingredients, supplements and drugs. Several examples are alpha-lioic acid, n-acetylcysteine (Farr SA, Poon HF, Dogrukol-Ak D, Drake J, Banks WA, Eyerman E, Butterfield DA, Morley JE (2003) The antioxidants α-lipoic acid and N-acetylcysteine reverse memory impairment and brain oxidative stress in aged SAMP8 mice. J Neurochemistry. 84, 1173-1183), polyunsaturated fatty acids (Petursdottir AL, Farr SA, Morley JE, Banks WA, Skuladottir GV (2008) Effect of dietary n-3 polyunsaturated fatty acids on brain lipid fatty acid composition, learning ability, and memory of senescence-accelerated mouse. J Gerontol A Biol Sci Med Sci 63, 1153-1160) and memantine (Zhou, M., Wang, M. -., & Wang, X. -. (2010). Effects of memantine combined with enriched environment therapy on learning and memory abilities and the mental behavior in senescence accelerated mouse. Journal of Clinical Neurology, 23(6), 438-441), all of which were demonstrated positive cognitive benefits in follow-up human clinical trials (Volchegorskii, I. A., Rassokhina, L. M., Kolyadich, M. I., & Alekseev, M. N. (2011). Comparative study of alpha-lipoic acid and mexidol effects on affective status, cognitive functions and quality of life in diabetes mellitus patients. Eksperimental'Naya i Klinicheskaya Farmakologiya, 74(11), 17-23; Chan, A., Remington, R., Kotyla, E., Lepore, A., Zemianek, J., & Shea, T. B. (2010). A Vitamin/nutriceutical formulation improves memory and cognitive performance in community-dwelling adults without dementia. Journal of Nutrition, Health and Aging, 14(3), 224-230; Small, G. W., Silverman, D. H. S., Siddarth, P., Ercoli, L. M., Miller, K. J., Lavretsky, H., Phelps, M. E. (2006). Effects of a 14-day healthy longevity lifestyle program on cognition and brain function. American Journal of Geriatric Psychiatry, 14(6), 538-545; Litvinenko, I. V., Odinak, M. M., Mogil'Naya, V. I., & Perstnev, S. V. (2010). Use of memantine (akatinol) for the correction of cognitive impairments in Parkinson's disease complicated by dementia. Neuroscience and Behavioral Physiology, 40(2), 149-155). Neuroscientists evaluating cognitive ingredients and/or drugs recognize that the SAMP8 is a valid animal model that has been successfully utilized for predicting their effectiveness in follow-up human clinical trials.
WO 2008/090474A describes methods and compositions for the production of food and nutraceutical substances comprising rosmarinic acid from spearmint plants and their use in treating allergic reactions and diseases.

### Summary of the Invention

The present invention relates to a water extract of a spearmint plant comprising 5% rosmarinic acid for use by oral administration in an amount of said extract between 0.01 and 50 mg/kg/day in therapeutically enhancing, improving or sustaining cognitive health and/or cognitive function in mammals experiencing normal aging cognitive changes.

Also provided is water extract of a spearmint plant comprising 5% rosmarinic acid for use by oral administration in an amount of said extract of between 0.01 and 50 mg/kg/day in the therapeutic maintenance of normal cognitive function under disruption of circadian rhythms as occurs in a condition selected from the list consisting of time zone changes, pregnancy, medications, changes in routine and shift work.

In another embodiment, the invention provides a water extract of a spearmint plant comprising 5% rosmarinic acid for use by oral administration in an amount of said extract of between 0.01 and 50 mg/kg/day in therapeutically treating or preventing a decline in cognitive health and/or cognitive function in a mammal experiencing normal aging cognitive changes.

The invention is defined by the claims. Any subject matter falling outside the scope of the claims is provided for information purposes only. Any references in the description to methods of treatment refer to the compounds, pharmaceutical compositions and medicaments of the present invention for use in a method of treatment of the human or animal body by therapy.

The invention consists of the administration of rosmarinic acid in a water extract of a spearmint plant as defined in the claims to improve learning, memory, executive function (i.e., working memory, task switching, planning, flexibility, visual attention, math skills and your ability to recognize and adapt to new and changing conditions, environments and situations), and cognitive associated behaviors (i.e., focus, attention, alertness, exploration, motivation, and the like) as defined in the claims.

Rosemary extract with carnosic acid shows improved declarative memory and procedural learning and memory. In mammals this may be observed as enhanced, improved or sustained memory of discrete events, places, people, and facts. In addition the ability to learn and retain habitual information or skills may be observed to improve, perhaps as a result of improved ability to make associations. Spearmint extract with rosmarinic acid shows improvement in declarative learning and memory. In mammals, this may be observed as enhanced or improved learning of discrete events, places, people, and facts along with enhanced and sustained memory of these things.
Oxidative damage is considered one of the hallmarks of the aging process. The neuronal dysfunction present in cognitive impairments associated with aging is thought in large part to be from oxidative stress. Both structural and functional damage to mitochondria is present in cognitive disorders, such as Alzheimer's disease, suggesting that antioxidants that penetrate both the cell and the mitochondria will provide the greatest protection from oxidative stress. The current study was designed to test if two novel, proprietary antioxidant-based ingredients, rosemary extract and spearmint extract standardized to carnosic acid and rosmarinic acid respectively, could improve learning and memory in the SAMP8 mouse model of accelerated aging. We have previously shown the ability of antioxidants to improve learning and memory deficits in the SAMP8 mice and reverse signs of oxidative damage; therefore, the SAMP8 mice are an excellent model to test these antioxidant compounds. SAMP8 mice were placed on one of three extracts. Two (comparative) rosemary extracts contained carnosic acid (60% carnosic acid and 10% carnosic acid) and one spearmint extract according to the invention contained 5% rosmarinic acid. Three doses of each extract were tested: 32 mg/kg, 16 mg/kg, 1.6 mg/kg of active (CA or RA, for rosemary and spearmint extracts, respectively) and vehicle control. A 50% SAMP8 backcross strain served as the control which also received vehicle. After 90 days of treatment mice were tested in 3 behavioral tests, T-maze foot shock avoidance, object recognition and lever press. Rosemary extract containing 60% carnosic acid improved acquisition at 32 mg/kg CA and retention at all three doses of active in T-maze foot shock avoidance, and was able to improve object recognition and lever press at 16 and 32 mg/kg CA. Rosemary extract with 10% carnosic acid improved retention in T-maze foot shock avoidance (16 mg/kg CA) and lever press at 16 and 32 mg/kg CA. Spearmint with 5% rosmarinic acid improved acquisition (16 and 32 mg/kg RA) and retention (at all doses of RA) in T-maze. In addition, spearmint with 5% rosmarinic acid improved object recognition at 16 and 32 mg/kg RA. 4-hydroxynonenal (HNE) was reduced in the brain cortex after treatment with all three extracts compared to the vehicle treated SAMP8. Protein carbonyls were reduced in the hippocampus after administration of rosemary with 10% carnosic acid and spearmint containing 5% rosmarinic acid. The current results indicate that the extracts from spearmint (rosmarinic acid) and rosemary (carnosic acid) have beneficial effects on learning and memory and brain tissue markers of oxidation that occur with age in SAMP8 mice.

### Brief Description of the Figures

Figures 1-8 are for reference.
Fig. 1 presents charts of the effect of 60% carnosic acid on T-maze acquisition, T-maze retention, object recognition and lever press.
Fig. 2 presents charts of the oxidative stress markers in the cortex of the brain following administration of rosemary extract containing 60% carnosic acid; the results are expressed in percent change from SAMP8 vehicle control; the * indicates P<0.05 and the **indicates P<0.01.
Fig. 3 presents charts of the oxidative stress markers in the striatum of the brain following administration of rosemary extract containing 60% carnosic acid; the results are expressed in percent change from SAMP8 vehicle control; the * indicates P<0.05 and the **indicates P<0.01.
Fig. 4 presents charts of the oxidative stress markers in the hippocampus of the brain following administration of rosemary extract containing 60% carnosic acid; the results are expressed in percent change from SAMP8 vehicle control; the * indicates P<0.05 and the **indicates P<0.01.
Fig. 5 presents charts of the effect of 10% carnosic acid on T-maze acquisition, T-maze retention, object recognition and lever press.
Fig. 6 presents charts of the oxidative stress markers in the cortex of the brain following administration of rosemary extract containing 10% carnosic acid; the results are expressed in percent change from SAMP8 vehicle control; the * indicates P<0.05 and the **indicates P<0.01.
Fig. 7 presents charts of the oxidative stress markers in the striatum of the brain following administration of rosemary extract containing 10% carnosic acid; the results are expressed in percent change from SAMP8 vehicle control; the * indicates P<0.05 and the **indicates P<0.01.
Fig. 8 presents charts of the oxidative stress markers in the hippocampus of the brain following administration of rosemary extract containing 10% carnosic acid; the results are expressed in percent change from SAMP8 vehicle control; the * indicates P<0.05 and the **indicates P<0.01.
Fig. 9 presents charts of the effect of 5% rosmarinic acid on T-maze acquisition, T-maze retention, object recognition and lever press.
Fig. 10 presents charts of the oxidative stress markers in the cortex of the brain following administration of rosemary extract containing 5% rosmarinic acid; the results are expressed in percent change from SAMP8 vehicle control; the * indicates P<0.05 and the **indicates P<0.01.
Fig. 11 presents charts of the oxidative stress markers in the striatum of the brain following administration of rosemary extract containing 5% rosmarinic acid; the results are expressed in percent change from SAMP8 vehicle control; the * indicates P<0.05 and the **indicates P<0.01.
Fig. 12 presents charts of the oxidative stress markers in the hippocampus of the brain following administration of rosemary extract containing 5% rosmarinic acid; the results are expressed in percent change from SAMP8 vehicle control; the * indicates P<0.05 and the **indicates P<0.01.

### Description of the Invention

The current study was designed to test if rosemary extract with CA (comparative) and spearmint extract with RA (according to the invention) could improve all facets of cognition health and/or cognitive functioning including, but not limited to: attention, alertness, recognition, recall, reaction time, focus, motivation, behavior, executive function, learning and memory. CA and RA molecules were specifically chosen due to their unique properties: 1) ability of these molecules to cross the blood brain barrier; 2) different solubility (oil vs. water); and 3) enhanced antioxidant capacity. The two extracts were tested in the SAMP8 mouse strain (a senescence accelerated mouse model). The SAMP8 mouse strain displays early onset of learning and memory deficits due to dementia resulting from beta amyloid plaque development and oxidative stress. The 50% SAM mouse served as the control.

### Definitions

As used in this application, the following terms have the meanings set out below.

Alertness: The state of paying close and continuous attention, being watchful and prompt to meet danger or emergency, or being quick to perceive and act.
Cognitive Health: Cognitive health refers to the health of the overall brain, tissues and blood supply as well as its' ability to function appropriately under various conditions. Good cognitive health is vital for the brain to perform all mental processes; collectively known as cognition including, but not limited to, learning, intuition, judgment, language, attention, alertness, focus and memory (both long and short-term); at peak performance. Poor cognitive health due to aging, diseases and/or other cognitive detriments reduce the brain's ability to function appropriately resulting in significant declines in cognitive function and performance.

Cognitive Function: Any mental or intellectual process involving neurological or symbolic operations including, but not limited, to communication, perception, comprehension, reasoning, memory, thinking, awareness, focus, attention, alertness, motivation, drawing conclusions, executive function, creation of imagery and capacity for judgment. In animal model systems, cognitive function may be measured in various conventional ways known in the art, including using a Morris Water Maze (MWM), Barnes circular maze, elevated radial arm maze, T-maze or any other mazes in which the animals use spatial information. Other tests known in the art may also be used to assess cognitive function, such as novel object recognition and odor recognition tasks.

Executive Function: Cognitive processes that regulate, control, and manage other cognitive processes, such as planning, working memory, attention, problem solving, verbal reasoning, mathematical ability, inhibition, mental flexibility, task switching, initiation, flexibility, visual attention, math skills, adaptability to new and changing environments and monitoring of actions.

Learning: The act, process, or experience of gaining knowledge or skill; psychological or behavioral modification especially through experience or conditioning.

Memory: The collection of information gained from past learning or experience that is stored in a person's mind. A piece of information, such as the mental image of an experience, that is stored in the memory. The ability to remember past experiences or learned information, involving advanced mental processes such as learning, retention, recall, and recognition and resulting from chemical changes between neurons in several different areas of the brain, including the hippocampus. Included are (1) declarative learning or memory, which refers to which can be consciously recalled such as facts and knowledge, (2) working memory, which refers to actively holding multiple pieces of transitory information in the mind where they can be manipulated, (3) reference memory, which refers to information gained from previous experience, either recent or remote, (4) recognition memory, which is the ability to recognize previously encountered events, objects, or people, and (5) associative memory, which is the ability to learn and remember the relationship between unrelated items. Each of these has and immediate, short-term, and long-term component. Immediate memory lasts for just a few seconds. Short-term memory stores information that has been minimally processed and is available only for a few minutes, as in remembering a phone number just long enough to use it. Short-term memory is transferred into long-term memory, which can last for many years, only when repeated use of the information facilitates neurochemical changes that allow it to be retained.

Therapeutically effective amount: The amount of a compound or composition or derivatives thereof of the present invention is an amount that, when administered to a subject, will have the intended therapeutic effect. The full therapeutic effect does not necessarily occur by administration of one dose, and may occur only after administration of a series of doses. Thus, a therapeutically effective amount may be administered in one or more administrations. The precise effective amount needed for a subject will depend upon, for example, the subject's size, health and age, the nature and extent of the cognitive impairment, and the therapeutics or combination of therapeutics selected for administration, and the mode of administration. The skilled worker can readily determine the effective amount for a given situation by routine experimentation. In one embodiment, the spearmint extract according to the invention is for administration on a daily frequency or more than once a day, e.g. 2, 3 or 4 times a day Treatment or Treating: Clinical intervention in an attempt to alter the natural course of the individual, animal or cell being treated, and may be performed either for prophylaxis or during the course of clinical pathology. Desirable effects include preventing occurrence or recurrence of disease, alleviation of symptoms, diminishment of any direct or indirect pathological consequences of the disease, lowering the rate of disease progression, amelioration or palliation of the disease state, and remission or improved prognosis. A condition or subject refers to taking steps to obtain beneficial or desired results, including clinical results. Beneficial or desired clinical results include, but are not limited to, enhancing, improving or sustaining cognitive health and/or function, alleviation or amelioration of one or more symptoms associated with mild cognitive impairment, or age-related cognitive impairment, delay or slowing of that impairment, amelioration, palliation or stabilization of that impairment, and other beneficial results, such as improvement of cognitive function or a reduced rate of decline of cognitive function in subjects with age-related cognitive impairment or at risk thereof

### EXAMPLE 1

Oxidative damage is considered one of the hallmarks of the aging process [Harman D (2002) Alzheimer's disease: role of aging in pathogenesis. Ann N Y Acad Sci. 959, 384-395]. The neuronal dysfunction present in diseases associated with aging such as Alzheimer's disease is thought in large part to be from oxidative stress [Markesbery WR (1997) Oxidative stress hypothesis in Alzheimer's disease. Free Radic Biol Med. 23, 134-147; Polidori MC, Griffiths HR, Mariani E, Mecocci P (2007) Hallmarks of protein oxidative damage in neurodegenerative diseases: focus on Alzheimer's disease. Amino Acids. 32, 553-559]. Both structural and functional damage to mitochondria is present in Alzheimer's disease suggesting that antioxidants that easily penetrate both the cell and the mitochondria will provide the greatest protection from oxidative stress [Skulachev VP, Anisimov VN, Antonenko YN, Bakeeva LE, Chernyak BV, Erichev VP, Filendo OF, Kalinia NI, Kapelko VI, Kolosova NG, Kopin BP, Korshunova GA, Lichinitser MR, Obukhova LA, Pasyukova EG, Pisarenko OI, Roginsky VA, Ruuge EK, Senin II, Severina II, Skulachev MV, Spivak IM, Tashlitsky VN, Tkachuk VA, Vyssokikh MY, Yaguzhinsky LS, Zorov DB (2009) An attempt to prevent senescence: a mitochondrial approach. Biochim Biophys Acta. 1787, 437-461; Suh JH, Shigeno ET, Morrow JD, Cox B, Rocha AE, Frei B, Hagen TM (2001) Oxidative stress in the aging rat heart is reversed by dietary supplementation with (R)-(alpha)-lipoic acid. FASEB J. 15, 700-706]. The senescence accelerated mouse-prone 8 (SAMP8) is a model of accelerated aging that develops deficits in learning and memory by 8 months of age [Yagi, et al., 1988; Flood, et al., 1998]. The SAMP8 mouse has a natural mutation leading to age-related increases in the amyloid precursor protein (AβPP) and amyloid beta (Aβ) in the brain, learning and memory deficits and oxidative stress. Furthermore, antisense to AβPP enhances memory and learning in these mice [Morley JE, Farr SA, Flood JF (2002) Antibody to amyloid beta protein alleviates impaired acquisition, retention, and memory processing in SAMP8 mice. Neurobiol Learn Mem. 78, 125-38; Kumar VB, Farr SA, Flood JF, Kamelesh V, Franko M, Banks WA, Morley JE (2000) Site-directed antisense oligonucleotide decreases the expression of amyloid precursor protein and reverses deficits in learning and memory in aged SAMP8 mice. Peptides. 21, 1769-1775]. In addition, SAMP8 mice have increased free radical production in the central nervous system [Butterfield, et al., 1997; Sato, et al., 1996] associated with mitochondrial dysfunction [Fujibayashi Y, Yamamoto S, Waki A, Jibusgu Hm Yonekura Y (1998) Increased mitochondrial DNA deletion in the brain of SAMP8, a mouse model for spontaneous oxidative stress brain. Neurosci Lett. 254, 109-112].

Antioxidants have been found to reverse learning and memory deficits in SAMP8 mice Alpha-lipoic acid and n-acetyl cysteine enhance memory and reverse indices of oxidative stress in these mice [Farr, et al., 2003]. Alpha-lipoic acid significantly decreased carbonyl levels of lactate dehydrogenase B, dihydro pyrimindase-like protein and alpha enolase [Poon HF, Farr SA, Thongboonkerd V, Lynn BC, Banks WA, Morley JE, Klein JB, Butterfield DA (2005) Proteomic analysis of specific brain proteins in aged SAMP8 mice treated with alpha-lipoic acid: implications for aging and age-related neurodegenerative disorders. Neurochem Int. 45, 159-68]. Polyphenols antioxidants have also been found to improve learning and memory in SAMP8 mice and reverse indices of oxidative stress [Farr SA, Price TO, Domnguez LJ, Motisi A, Saiano F, Niehoff ML, Morley JE, Banks WA, Ercal N, Barbagallo M (2012) Extra virgin olive oil improves learning and memory in SAMP8 mice. J Alzheimer's Dis 28, 81-92].

Carnosic acid (CA) and rosmarinic acid (RA) have been found to be neuroprotective and preventative against oxidative stress [Fadel O, El Kirat K, Morandat S (2011) The natural antioxidant rosmarinic acid spontaneously penetrates membranes to inhibit lipid peroxidantion in situ. Biochim Biophys Acta 1808, 2973-2980; Fallarini S, Miglio G, Paoletti T, Minassi A, Amoruso A, Bardelli C, Brunelleschi S, Lombardi G (2009) Clovamide and rosmarinic acid induce neuroprotective effects in invitro models of neuronal death. Br J Pharmacol 157, 1072-1084; Hou CW, Lin YT, Chen YL, Wang YH, Chou JL, Ping LY, Jeng KC (2012) Neuroprotective effects of carnosic acid on neuronal cells under ischemic and hypoxic stress. Nutr Neurosci [Epub]]. Carnosic acid also has an anti-inflammatory effect after LPS administration [19]. Protection against oxidative stress and inflammation has been associated with improved memory in diseases of aging [Farr, et al., 2012]. Rosmarinic acid improved memory in the Morris water maze spatial task [Park DH, Park SJ, Kim JM, Jung WY, Ryu JH (2010) Subchronic administration of rosmarinic acid, a natural prolyl oligopeptidase inhibitor, enhances cognitive performance. Fitoterapia 81, 644-648].

Memory is divided in to two main categories declarative (or explicit memory) and procedural (or implicit memory). Declarative memory is further subdivided into semantic (facts or meaning) and episodic (specific experiences). Semantic memory is generally derived from episodic memory. Declarative memories are thought of as being encoded by the hippocampus whereas procedural memories are thought of as being encoded by the caudate a structure within the striatum. Procedural or implicit memory comes from learning the association between a response and a reward. Procedural memories often start as declarative memories until they become ingrained or a habit.

T-maze foot shock avoidance task represents declarative episodic memory. It has temporal, spatial and associative memory components. The mice must learn to associate a buzzer and the door opening with the onset of shock [Farr SA, Banks WA, La Scola ME, Flood JF, Morley JE (2000) Permanent and temporary inactivation of the hippocampus impairs T-maze footshock avoidance acquisition and retention. Brain Res. 872, 242-249]. They must also learn if they turn left or right to escape or avoid the shock. Finally, it has a temporal component in that they learn that upon the opening of the door and simultaneous onset of the buzzer they have 5 sec to run to the goal box (on the left or right at the end of the corridor) to avoid the shock.

Object recognition memory is a form of declarative or explicit memory. It is the ability to recognize previously encountered events, objects or people [Medina JH, Bekinschtein P, Commarota M, Izquierdo I (2008) Do memories consolidate to persist or do they persist to consolidate? Behav Br Res 192:61-69]. In this task the mice that remember the previous objects they have seen will spend a greater amount of time exploring the new or novel object in the retention test. This test is also a hippocampal test. Studies have shown that lesions within the hippocampus result in deficits in memory 24 hours after the first exposure [Hammond RS, Tull LE, Stachman RW (2004) On the delay-dependent involvement of the hippocampus in object recognition. Neurbiol Learn Mem 82, 26-34]. Human tests evaluating recognition show subjects word lists or objects and then, upon retention testing, switch some of the words or objects and ask the subjects to identify the ones they have previously encountered.

The lever press is an operant task. The mice learn to associate the pressing of the lever with a food reward. Initially, this test utilizes declarative memory to form the association but once the association has been formed it becomes a procedural task involving primarily the caudate region of the brain [Beninger RJ, Ranaldi R (1993) Microinjectiosn of flupenthixol into the caudate-putamen but not the nucleus accumbens, amygdala or frontal cortex of rats produce intra-session declines in food-rewarded operant responding Behav Brain Res 55, 203-212]. Sometimes the lever press is referred to as a Skinner box after the name of its inventor.

The current study was designed to test if these two novel antioxidants found in rosemary extract and spearmint extract, carnosic acid and rosmarinic acid respectively, could improve learning and memory in the SAMP8 mouse model of accelerated aging. We have previously shown the ability of antioxidants to improve learning and memory deficits in the SAMP8 mice and reverse signs of oxidative damage [Farr, et al., 2003; Morley JE, Armbrecht HJ, Farr SA, Kumar VB (2012) The senescence accelerated mouse (SAMP8) as a model for oxidative stress and Alzheimer's disease. Biochim Biophy Acta 1822]; therefore, the SAMP8 mice are an excellent model to test these antioxidant compounds [Farr SA, Yamada KA, Butterfield DA, Abdul HM, Xu L, Miller NE, Banks WA, Morley JE (2008) Obesity and hypertriglyceridemia produce cognitive impairment. Endocrinology 149, 2628-2636].

### Materials and Methods

### Mice.

At the start of treatment, the subjects for the experiments were 9 month old SAMP8 mice. The control group was 9 month old 50% SAM mice, a cross between a male SAMP8 and a female CD-1 mouse that does not show age-related impairment in learning and memory. These studies were conducted with the approval of the Animal Care and Use Committee at the VA Medical Center, St. Louis, MO.

### Treatment.

Nine-month old SAMP8 mice received rosemary extract containing either 60% or 10% CA (in sunflower oil) or spearmint extract containing 5% RA (in water) for 12 weeks via oral gavage once per day.

Mice were set up in 7 groups:
1. SAMP8 administered rosemary extract containing 60% CA (CA60);
2. SAMP8 administered rosemary extract containing 10% CA (CA10);
3. SAMP8 administered spearmint extract containing 5% RA (RA5) (according to the invention);
4. SAMP8 administered sunflower oil as a negative control for groups 1 and 2;
5. SAMP8 administered water as a negative control for group 3;
6. SAMP 50% backcross administered sunflower oil as a positive control for groups 1 and 2; and
7. SAMP 50% backcross administered water as a positive control for group 3.

Dose-response curves were conducted for each extract tested. Doses evaluated were 1.6 mg/kg, 16 mg/kg, 32 mg/kg of the active (either CA or RA, from rosemary and spearmint, respectively) and the vehicle groups for each of the three extracts listed above. Body weights were recorded weekly throughout the study and behavioral testing began in the twelfth week of feeding.

### Behavioral Testing.

Behavioral testing was started in the twelfth week of treatment. Mice were first trained in T-maze foot shock avoidance during week 12 (T-maze acquisition) and T-maze retention of this task was tested in week 13, followed by object recognition in week 13. Lever press was performed during weeks 14 and 15.

### T-Maze training and testing procedures

The T-maze is both a learning task based on working-memory and a declarative reference-memory task. The T-maze consisted of a black plastic alley with a start box at one end and two goal boxes at the other ends of the arms of the T-shape. The start box was separated from the alley by a plastic guillotine door that prevented movement down the alley until it was raised at the onset of training. An electrifiable floor of stainless steel rods ran throughout the maze to deliver a mild scrambled foot-shock.

Mice were not permitted to explore the maze prior to training. Training trials began when a mouse was placed into the start box, the guillotine door was raised and a cue buzzer sounded simultaneously; 5 sec later foot-shock was applied. The arm of the maze entered by the mouse on the first trial was designated the "incorrect" box and the mild foot-shock was continued until the mouse entered the other goal box. This goal box was designated as "correct" for the particular mouse in all subsequent trials. At the end of each trial, the mouse was returned to its home cage until the next trial.

Mice were trained until they made one avoidance (T-maze acquisition). Training was conducted with 35 sec intervals between trials, the buzzer was of the door-bell type sounded at 55 dB, and shock was set at 0.35 mA (Coulbourn Instruments scrambled grid floor shocker model E13-08). T-maze retention was tested one week later by continuing training. The results were reported as the number of trials to criterion for the retention test. Criterion is defined as avoiding the mild foot-shock 5 times in 6 consecutive attempts.

### Object -Place Recognition

Object-place recognition is a declarative memory task that involves the hippocampus when, as performed here, the retention exposure interval is 24 hours after initial exposure to the objects [Farr, et al., 2012]. Mice were habituated to an empty apparatus for 5 minutes a day for 3 days prior to entry of the objects. During the training session, the mouse was exposed to two similar objects (plastic frogs) which it was allowed to examine for 5 minutes. The apparatus and the objects were cleaned between each mouse. Twenty-four hours later, the mouse was exposed to one of the original objects and a novel object in a new location. The percentage of time spent examining the objects was recorded. The novel object was made out of the same material as the original object and of the same size, but a different shape. This eliminated the possibility of the smell associated with a particular object being a confounding factor. The underlying concept of the task is based on the tendency of mice to spend more time exploring new, novel objects rather than familiar objects. Thus, the greater the retention/memory at 24 hours, the more time spent with the new object.

### Lever Press for Milk Reinforcement

Lever press is a procedural (operant) associative learning and memory task. Mice were placed into a fully automated lever press chamber. Pressing a lever on one wall of the compartment caused a light and liquid dipper with 100 µl of milk to appear on the opposite wall. On day 1 the mice had 11 sec to obtain the reward; on all subsequent days, mice had 6 sec obtain the reward. Mice were given 40 min training sessions on M, W, F for two weeks. Mice were food deprived 16 hours prior to the start of the test to provide motivation to perform the task. Food was returned immediately upon completion of a session. Acquisition was measured as the number of rewarded lever presses [Farr SA, Yamada KA, Butterfield DA, Abdul HM, Xu L, Miller NE, Banks WA, Morley JE (2008) Obesity and hypertriglyceridemia produce cognitive impairment. Endocrinology 149, 2628-2636].

### Brain Oxidative Stress

### Sample Preparation

Brain samples were briefly homogenized with a Wheaton tissue homogenizer in an ice-cold lysis buffer (pH 7.4) containing 320 mM sucrose, 1% mM Tris-HCl (pH 8.8), 0.098 mM MgC12, 0.076 mM EDTA, and proteinase inhibitors leupeptin (0.5 mg/mL), pepstatin (0.7 µg/ml), aprotinin (0.5 mg/ml) and PMSF (40 µg/ml) and a phosphatase inhibitor cocktail. The homogenized samples were then diluted 2X with lysis buffer. After homogenization, a small aliquot of homogenized samples were sonicated for 10 seconds at 20% power with a Fisher 550 Sonic Dismembrator (Pittsburgh, PA, USA) and frozen. The remaining homogenate was centrifuged at 3000 g for 5 min and the supernatant cytosolic and membranous fractions were transferred out into another set of tubes. Following the addition of 400 µl of lysis buffer, the remaining pellet nuclear fraction was centrifuged at 3,000 g for 5 min and supernatant removed. The pellet was suspended in 20 µl of lysis buffer and inhibitor. The supernatant cytosolic and membranous fractions were centrifuged at 10,000 g for 10 min, and the resulting supernatant cytosolic fraction was transferred out into another set of tubes leaving the pellet membranous fraction. All sonicated samples and fractions were stored at -70 °C until used for further experiments. Protein concentrations were measured through Pierce Bicinchoninic Acid (BCA)

### Slot blot analysis

### Protein Carbonyls

For protein carbonyl detection, slot blot analysis of the 2,4-dinitrophenyl hydrazone (DNP) schiff-base adduct of the carbonyls was employed. Sample aliquots of 5 µl were incubated at room temperature with 5 µl of 12% sodium dodecyl sulfate and 10 µl of 2,4-dinitrophenylhydrazine (from OxyBlottm Protein oxidation kit, Chemicon-millipore, Billerica, MA, USA) for 20 min, followed by the addition of 7.5 µl of neutralization solution containing Tris (2 M) in 30% glycerol to each sample. Following derivatization samples were diluted to 1 µg/mL using 1x phosphate buffer solution (PBS) containing sodium chloride, mono, and dibasic sodium phosphate. The corresponding sample solutions (250 µl) were rapidly loaded as duplicates onto a nitrocellulose membrane through water vacuum pressure. The resulting protein-bound nitrocellulose membrane was then blocked with fresh blocking solution containing 750mg of bovine serum albumin (BSA) in 25ml of wash blot containing 35.2 g sodium chloride, 1.77 g monobasic sodium phosphate, 9.61 g dibasic sodium phosphate and 1.6 mL TWEEN, diluted to 4 L with deionized water for 90 min. The membrane was then incubated with polyclonal RbxDNP (from OxyBlottm Protein oxidation kit, Chemicon-millipore, Billerica, MA, USA, dilution 1:100) in wash blot for 2 h. After three 5 min washes with fresh wash blot, the membrane was then incubated with polyclonal anti-rabbit IgG alkaline phosphatase (Chemicon, Temecula, CA, USA, dilution 1:8000) for 1 hour and washed with fresh wash blot in three increments of 5, 10 and 10 min. After washing, the membrane was developed colorimetrically using a 5-bromo-4-chloro-3-indolyl-phosphate/nitroblue tetrazolium reagent solution for alkaline phosphatase secondary antibody. After development, blots were dried and scanned on a CanoScan8800F (Canon) scanner using Adobe Photoshop and analyzed using Scion Image software (Scion Corporation).

### 4-Hydroxy-2-trans-noneal (HNE)

Levels of protein-bound HNE are used as a marker of lipid peroxidation and were determined as previously described [29]. For slot blot analysis of protein-bound HNE detection, sample aliquots of 5 µl were incubated at room temperature with 5 µl of 12% sodium dodecyl sulfate and 10 µl of Laemmli buffer for 20 min, followed by dilution to 1 µg/ml using 1x phosphate buffer solution (PBS) containing sodium chloride, mono, and dibasic sodium phosphate. The corresponding sample solutions (250 µl) were rapidly loaded as duplicates onto a nitrocellulose membrane through water vacuum pressure. The resulting protein-bound nitrocellulose membrane was then blocked with fresh blocking solution for 90 min. The membrane was then incubated with polyclonal anti-HNE (Alpha diagnostic, San Antonio, TX, USA, dilution 1:5000) in wash blot for 2 h. After three 5 min washes with fresh wash blot, the membrane was then incubated with polyclonal anti-rabbit IgG alkaline phosphatase (Chemicon, Temecula, CA, USA, dilution 1:8000) for 1 hour and washed with fresh wash blot in three increments of 5, 10 and 10 min. After washing, the membrane was developed colorimetrically using a 5-bromo-4-chloro-3-indolyl-phosphate/nitroblue tetrazolium reagent solution for alkaline phosphatase secondary antibody. After development, blots were dried and scanned on a CanoScan8800F (Canon) scanner using Adobe Photoshop and analyzed using Scion Image software (Scion Corporation).

### 3-Nitrotyrosine (3-NT)

3-NT levels were used as an additional marker of protein oxidative damage [Suh, et al., 2001; Butterfield DA, Stadtman ER (1997) Protein oxidation processes in aging brain. Adv Cell Aging Gerontol 2, 161-191]. Samples (5 µL) were incubated at room temperature for 20 min in 5 µL of 12% SDS and 10 µl of Laemmli buffer (0.125 M Trizma base, 4% SDS, 20% glycerol),for 20 min. . Samples (250 ng of protein) per slot were blotted onto a nitrocellulose membrane. A primary rabbit antibody (Sigma-Aldrich) specific for 3-NT (1:1000) was used. The same secondary goat anti-rabbit (Sigma-Aldrich) antibody was then used for detection of each primary antibody. Blots were developed and quantified as described above for protein carbonyls. The developing and detection were perform as described above for protein carbonyls.

### Triglycerides

Serum triglyceride was quantitated using an enzymatic assay system from Pointe Scientific, Inc. (Canton, MI) which incorporated a linear, endpoint color reaction. Triglycerides in the sample are hydrolyzed by lipase to glycerol. The glycerol is then phosphorylated by glycerol kinase and ATP to glycerol-3-phosphate (G₃P) and ADP. The G₃P is converted to dihydroxyacetone phosphate (DAP) and hydrogen peroxide. The hydrogen peroxide reacts with 4-aminoantipyrine (4-AAP) and 3-hydroxy-2,4,6-tribomobenzoic acid (TBHB) in a reaction catalyzed by peroxidase to yield a red colored quinoneimine dye. The intensity of color produce was measured at 540nm using a Bio-Rad microplate reader (Hercules, CA).

### Statistics

Results were analyzed using analysis of variance (ANOVA) to examine the difference among groups. The measure of acquisition and retention in the T-maze were the number of trials to reach criterion. The results for object recognition are presented in percentage of time spent exploring the novel object out of total exploration time. Lever press was analyzed by a two-way repeated measures ANOVA. Results are expressed as means plus/minus standard errors. Tukey's or Bonferroni's post hoc analysis was used to compare means between groups. Dunnett's was used for comparison to the SAMP8 Vehicle control group. The brain tissue oxidative stress parameters were analyzed using a Mann-Whitney U test.

### Results

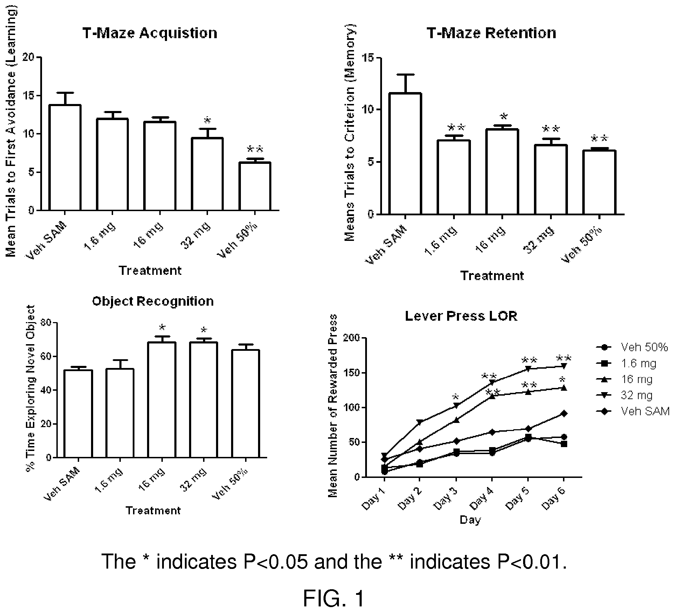

*Comparative Rosemary Extract containing 60% CA (CA60):* The results discussed below are shown in Fig. 1.

### Behavioral testing

T-maze: The one-way ANOVA for trials to criterion on the T-maze acquisition test showed a significant treatment effect F(4, 48) 8.98, p<0.001. Bonferroni's post hoc test indicated that the SAMP8 mice that received 32 mg/kg CA took significantly fewer trials to reach criterion than the mice that received vehicle. In addition, the mice that received 32 mg/kg CA were not significantly different from the 50% SAM control mice. The one-way ANOVA for trials to criterion on the T-maze retention test showed a significant treatment effect F(4,47) = 7.25, p<0.001. Bonferroni's post hoc analysis indicated that the SAMP8 mice that received 32, 16 and 1.6 mg/kg CA took significantly fewer trials to reach criterion than the SAMP8 mice that received vehicle. The mice that received 32, 16 and 1.6 mg/kg CA were not significant different from the 50% SAM mice.

Object Recognition: The one-way ANOVA for time spent exploring the novel object on the 24 hour retention test produced a significant treatment effect F(4,46) = 4.88, p<0.003. Bonferroni's post hoc test indicated that the SAMP8 mice which received 32 and 16 mg/kg CA spent significantly greater amount of time exploring the novel object than the SAMP8 mice which received vehicle. The SAMP8 mice which received 32 and 16 mg/kg CA were not significantly different from the 50% SAM controls.

Lever Press: The two-way repeated measures ANOVA, for treatment and day, evaluation of the number of rewarded lever presses produced a significant effect for treatment F(4,257) = 17.27, P<0.001 and day F(5,257) = 15.31, P<0.001. The interaction treatment x day was not significant F(20,257) = 3.78, NS. Tukey's post hoc analysis indicated that on days 3, 4, 5 and 6,

SAMP8 mice which received 32 mg/kg CA received significantly more rewards than the SAMP8 mice which received the vehicle control. The SAMP8 mice which received 16 mg/kg CA received significantly more rewards on days 4, 5, and 6 compared to the SAMP8 mice which received the vehicle control.

### Triglyceride Levels

The one-way ANOVA for triglyceride levels was not significant F(4,50) = 2.42 for mice administered CA60.

### Brain Oxidative Stress

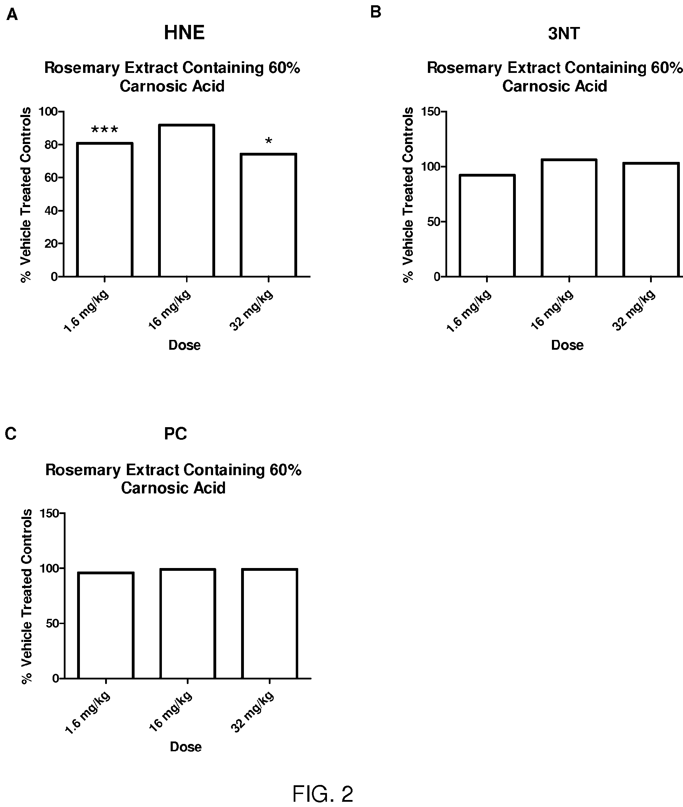

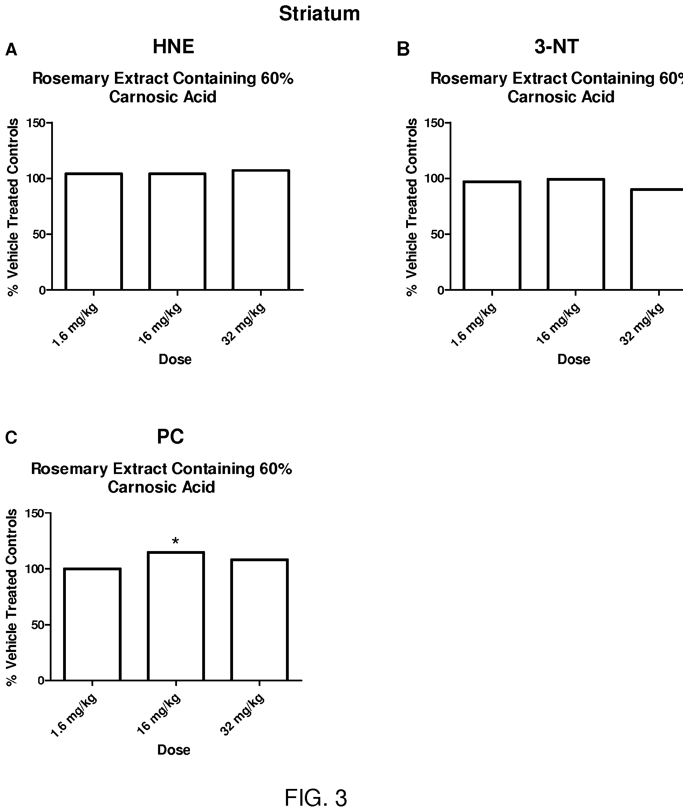

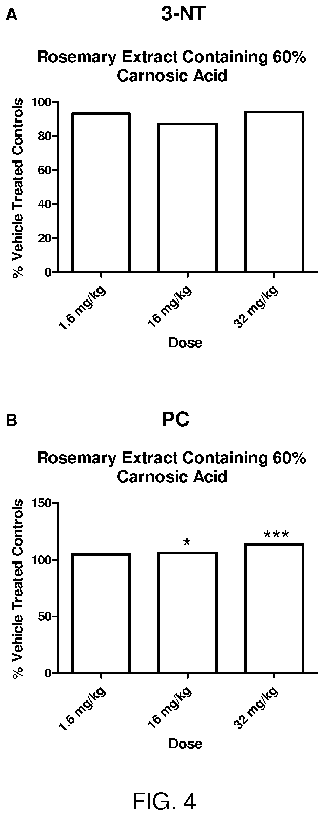

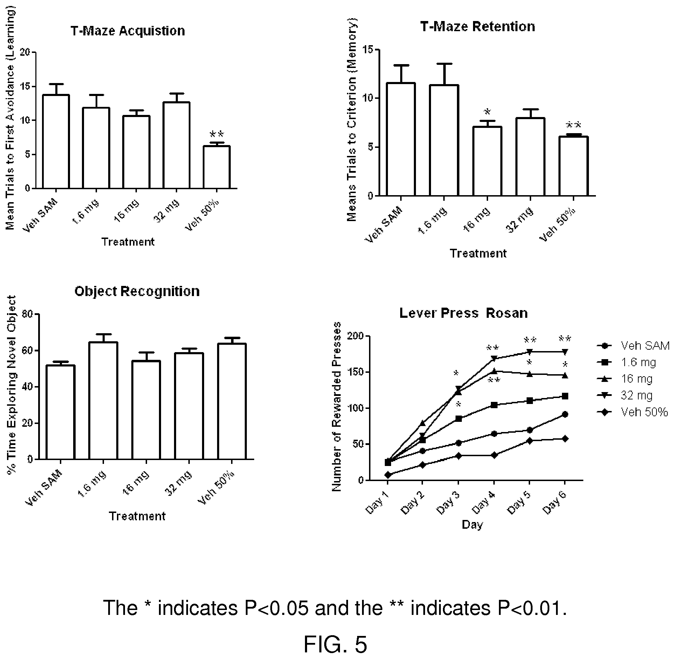

Mann-Whitney U test indicated that CA60 significantly decreased 4-hydroxlnonenal (HNE) in the cortex at 1.6 and 32 mg/kg CA in comparison to SAMP8 administered sunflower oil as a vehicle control. There was no significant effect of CA60 on 3-nitrotyrosine (3-NT) or protein carbonyls within the cortex (See Figure 2). CA60 significantly increased protein carbonyls in the striatum at 16 mg/kg CA, but had no effect on HNE or 3-NT (See Figure 3). CA60 significantly increased protein carbonyls in the hippocampus at 16 mg/kg CA. CA60 had no effect on 3-NT in the hippocampus (See Figure 4).
*Comparative Rosemary Extract containing 10% CA (CA10):* The results discussed below are shown in Fig. 5.

### Behavioral Testing.

T-maze: The one-way ANOVA for trials to criterion during T-maze acquisition produced a significant effect for group F(4, 44) F=5.914, p<0.001. Bonferroni's post hoc test indicated that the 50% SAM mice that received vehicle took significantly fewer trials to reach criterion than the SAMP8 mice which received 32, 1.6 mg/kg CA or vehicle. The mice that received 16 mg/kg CA were not significantly different from the 50% SAM which received vehicle or the SAMP8 mice that received vehicle. The ANOVA for trials to criterion on the T-maze retention test indicated a significant effect of treatment F(4, 44) = 4.04, p<0.007. Dunnett's post hoc test indicated that the mice which received 16 mg/kg CA took significantly fewer trials to reach criterion than the SAMP8 mice which received vehicle.

Object Recognition: The one-way ANOVA for time spent exploring the novel object was not significant F(4, 44) = 2.249; p<0.08.

Lever Press: The two-way repeated measures ANOVA, for treatment and day, for evaluation of number of rewarded lever presses produced a significant effect for treatment F(4,253) = 6.74, P<0.001 and day F(5,253) = 7.53, P<0.001. The interaction treatment x day was not significant F(20,253) = 1.83. Tukey's post hoc analysis indicated that on days 3, 4, 5 and 6, mice, which received 32 mg/kg and 16 mg/kg CA received significantly more rewards than the mice which received vehicle.

### Triglyceride Levels

The one-way ANOVA for triglyceride levels indicated a significant effect F(4,47) = 3.11, P<0.02 following administration of CA10. Tukey's post hoc test indicated that the 50% SAM mice had significantly higher triglyceride levels than the SAMP8 mice that received 1.6 mg/kg CA. There were no other significant differences between any of the other groups.

### Brain Oxidative Stress

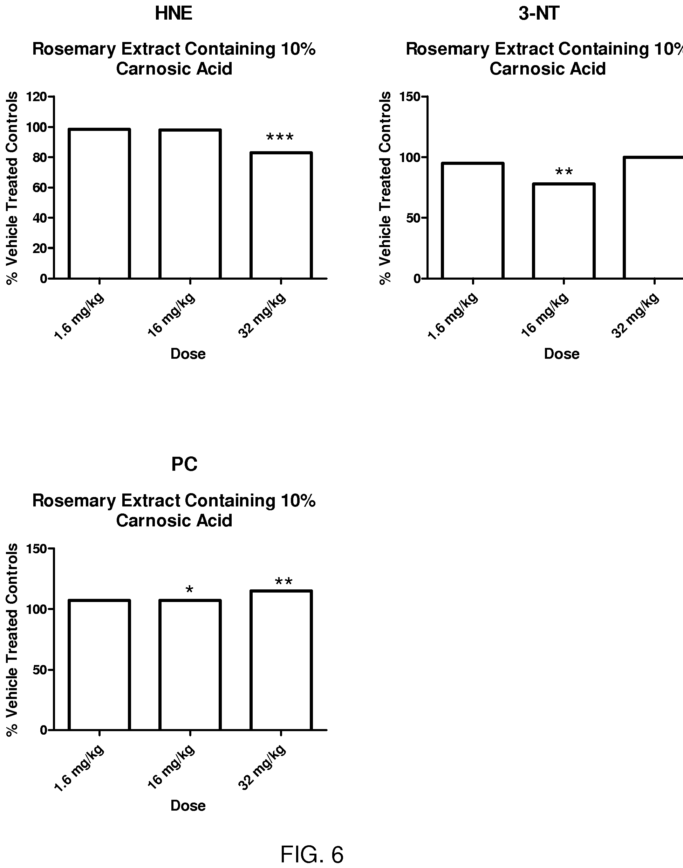

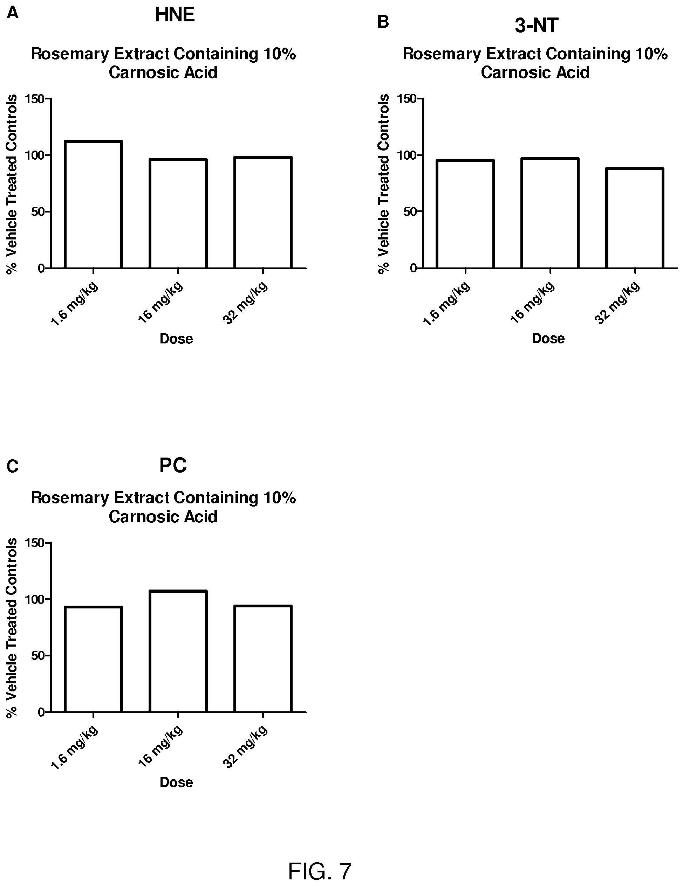

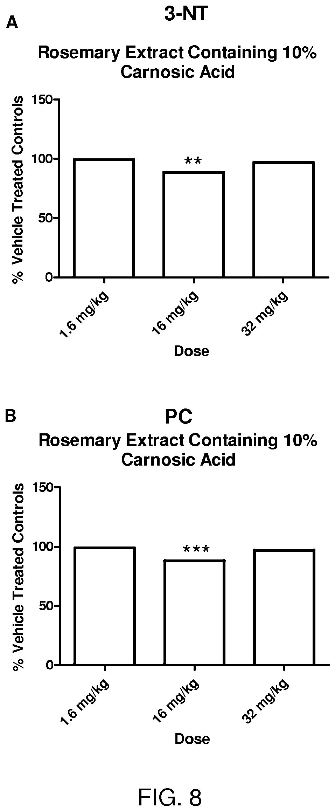

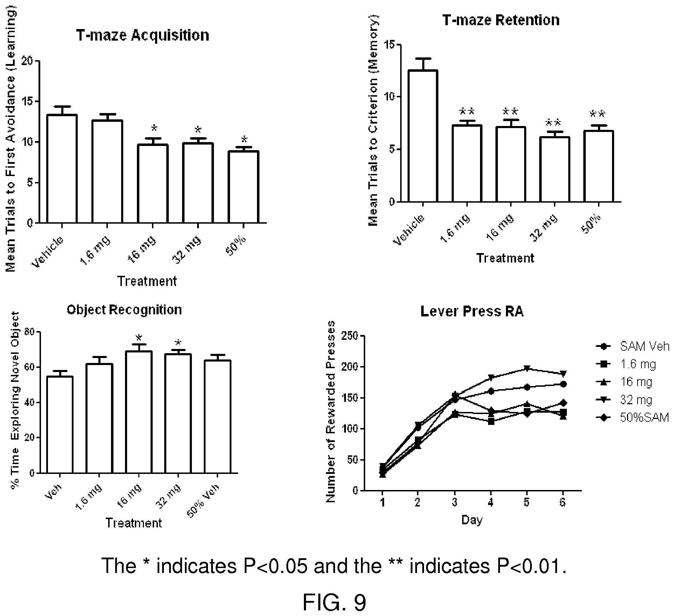

The Mann-Whitney U test indicated that CA10 significantly decreased HNE in the cortex at 32 mg/kg CA, 3-NT in the cortex at 16 mg/kg CA and significantly increased protein carbonyls at 16 and 32 mg/kg CA (See Figure 6) in comparison to SAMP8 vehicle treated controls. In addition, CA10 had no effect on HNE, 3-NT or protein carbonyls in the striatum (See Figure 7). Finally, CA10 significantly decreased 3-NT and protein carbonyls at 16 mg/kg CA in the hippocampus (See Figure 8) compared to vehicle treated SAMP8 controls.
*Spearmint Extract Containing 5% RA (RA5):* The results discussed below are shown in Fig. 9.

### Behavioral Testing.

T-maze: The one-way ANOVA for trials to criterion on the T-maze acquisition test showed a significant treatment effect F(4, 52) 6.38, p<0.001. Bonferroni's post hoc test indicated that the SAMP8 mice that received 32 and 16 mg/kg RA took significantly fewer trials to reach criterion than the mice that received vehicle. The one-way ANOVA for trials to criterion on the T-maze retention test showed a significant treatment effect F(4,50) = 12.77, p<0.001. Bonferroni's post hoc analysis indicated that the SAMP8 mice that received 32, 16 and 1.6 mg/kg RA took significantly fewer trials to reach criterion than the SAMP8 mice that received vehicle. The mice that received 32, 16 and 1.6 mg/kg RA were not significantly different from the 50% SAM control mice.

Object Recognition: The one-way ANOVA for time spent exploring the novel object produced a significant treatment effect F(4,47) = 2.79; p<0.03. Dunnett's post hoc test indicated that the mice which received 32 and 16 mg/kg RA spent significantly greater amount of time investigating the novel object than the SAMP8 mice which received vehicle.

Lever Press: The two-way repeated measures ANOVA, for treatment and day, for evaluation of the number of rewarded lever presses produced a significant effect for treatment F(4,257) = 6.18, P<0.001 and day F(5,257) = 40.98, P<0.001. The interaction treatment x day was not significant F(20,257) = 2.44. Tukey's post hoc analysis indicated there was no significant difference between the SAMP8 mice that received RA5 and the SAMP8 mice that received vehicle.

### Triglyceride Levels

The one-way ANOVA for triglyceride levels after treatment produced a significant effect F(4, 44) = 4.06, P<0.006. Tukey's post hoc analysis indicated that the 50% SAM control mice had significantly higher triglyceride levels compared to the SAMP8 mice that received vehicle and the SAMP8 mice that received 1.6 mg/kg RA. There were no other differences between groups.

### Brain Oxidative Stress

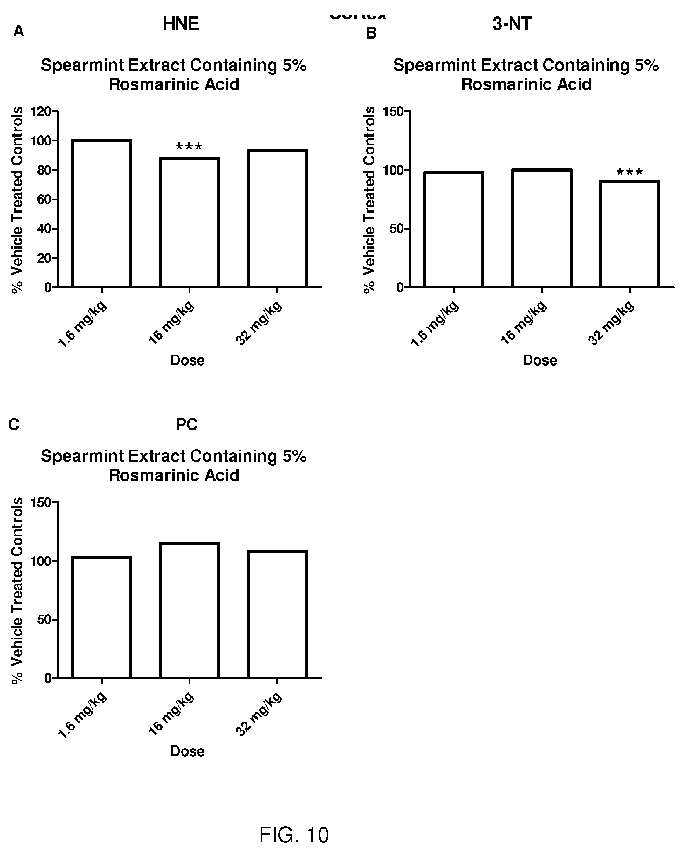

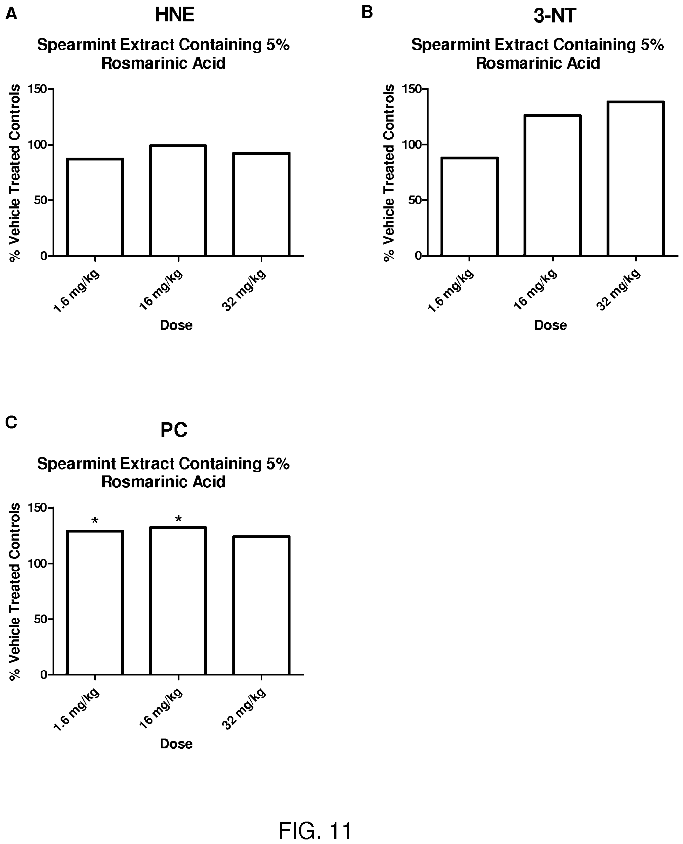

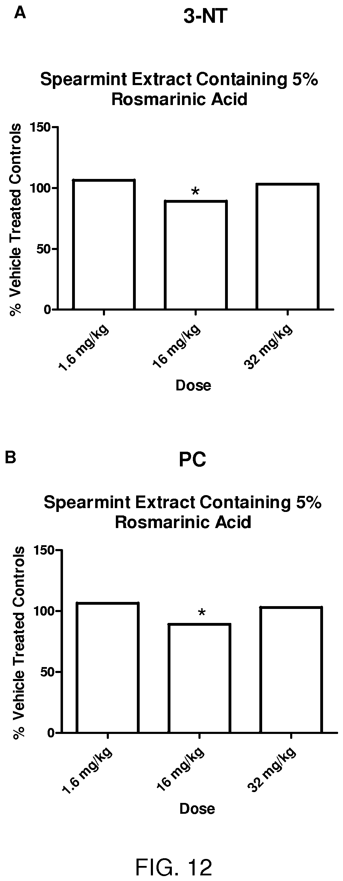

Mann-Whitney U test indicated that RA5 significantly decreased HNE in the cortex of mice that received 16 and 32 mg/kg RA and decreased 3-NT at 32 mg/kg RA. RA5 had no effect on protein carbonyls in the cortex (See Figure 10). In addition, RA5 had no effect on HNE or 3-NT within the striatum. Mice that received RA5 had significantly higher protein carbonyl levels at 1.6 and 16 mg/kg RA compared to the vehicle treated control SAMP8 mice (See Figure 11) within the striatum. RA5 significantly reduced levels of 3-NT and protein carbonyls in the hippocampus at 16 mg/kg RA (See Figure 12)

### Discussion:

In the current study the extracts containing CA or RA improved learning and memory. Overall, CA had an effect on both declarative and procedural memory. The T-maze and object recognition both tested declarative memory, while the operant lever press tested procedural learning and memory. We found that comparative rosemary extract containing 60% CA improved in both declarative and procedural learning and memory . Comparative rosemary extract with 10% CA improved declarative memory and both procedural learning and memory as evaluated in the lever press operant task. Spearmint extract with 5%RA improved both declarative learning and memory. None of the compounds had an effect on body weight.

CA and RA both worked in a dose-response fashion. This is not surprising as most memory enhancing compounds demonstrate hormesis, the phenomena where there is an optimal dose for memory enhancement below which the compound in not effective and above which the compound produces impairment, thus producing what is known as an "inverted-U" shaped dose-response curve. This was previously reported in water maze testing with rosmarinic acid and in its anxiolytic ability as tested in an elevated plus maze [Park, et al., 2010; Butterfield, et al., 1997]. This previous study used a "stress" condition in a normal healthy, non-aging mouse model and found a cognitive-enhancing capability. Rosmarinic acid protected against Aβ toxicity in mice injected with Aβ [Pereira P, Tysca D, Oliveria P, da Silva Brum LF, Picada JN, Ardenghi P (2005) Neurobehavioral and genotoxic aspects of rosmarinic acid. Pharmacol Res 52, 199-203]. In the current study, we found that rosmarinic acid prevented memory in a mouse model that naturally overproduces Aβ.

The findings in the current study are similar to previous findings following antioxidant supplementation in SAMP8 mice. Alpha lipoic acid improved learning and memory following only one-week of treatment in 12 month old SAMP8 mice and after just 2 weeks in 18 month old SAMP8 mice [Farr, et al., 2003; Pereira, et al., 2005]. The antioxidant docosahexaenic acid, found in fish oil, also improved learning and memory in SAMP8 mice [Alkam, et al., 2007]. Supplementation of mulberry extract, rich in the antioxidant anthocyanin, resulted in improved avoidance learning and memory, reduced cholesterol and reduced indices of oxidative stress in SAMP8 mice [Farr SA, Price TO, Banks WA, Ercal N, Morley JE (2012) Effect of Alpha-Lipoic Acid on Memory, Oxidation and Lifespan in SAMP8 Mice. J Alzheimer's Dis (In Press**)**]. In addition, these findings are encouraging as several of the above mentioned molecules/extracts have demonstrated positive effects in follow-up human clinical trials for cognition, indicating the predictive and translatable nature of this model from rodent to human [Petursdottir, et al., 2008; Shih PH, Chan YC, Liao JW, Wang MF, Yen GC (2010) Antioxidant and cognitive promotion effects of anthocyanin-rich mulberry (Morus atropurpurea L.) on senescence-accelerated mice and prevention of Alzheimer's disease. JNutr Biochem 21, 598-605].

Protein and lipid oxidation occurs in SAMP8 mouse brains with age [Butterfield, et al., 1997; Poon, et al., 2005; Pereira, et al., 2005]. Proteins and lipids play important roles in the normal structure and function of cells [Butterfield DA, Reed T, Sultana R (2011) Roles of 3-nitrotyrosine- and 4-hydroxynonenal-modified brain proteins in the progression and pathogensis of Alzheimer's disease. Free Radic Res 45, 59-72]. Abnormal cell function and eventual cell death can occur with oxidative modification of proteins in cells [Lee HC, Wei YH (2012) Mitochondria and aging. Adv Exp Med Biol 942, 311-327]. In the present study, sensitive immunochemical methods were used to determine if treatment with the antioxidants had any effect on protein carbonyl levels. Our results indicate that a decrease in protein oxidation in the hippocampus (following administration of rosemary extract with 10% CA or the spearmint extract with 5% RA) and lipid oxidation in the cortex (following administration of all extracts). The hippocampus and cortex have been found to be important areas for T-maze learning and memory [Farr SA, Banks WA, La Scola ME, Flood JF, Morley JE (2000) Permanent and temporary inactivation of the hippocampus impairs T-maze footshock avoidance acquisition and retention . Brain Res 872, 242-249; Farr SA, Uezu K, Creonte TA, Flood JF, Morley JE (2000) Modulation of memory processing in the cingulate cortex of mice. Pharmacol Biochem Behav 65, 363-368]. In addition, studies have found that the hippocampus is important for memory in object recognition when using a 24 hour retention delay [Hammond, et al., 2004]. Our data show that both rosemary extract with carnosic and spearmint extract with rosmarinic acid can help in reversing oxidative changes that occur with aging and cognitive decline in SAMP8 mice.

In the current study, the antioxidants rosemary extract with carnosic and spearmint extract with rosmarinic acid were orally supplemented to determine if they had beneficial effects on learning and memory in the SAMP8 mouse model of the cognitive dysfunction. The extracts resulted in an inverted U-shaped dose-response curve as demonstrated by most memory enhancing compounds and efficacious doses of both extracts were identified. The extracts tested demonstrated beneficial effects in both declarative and procedural memory, both of which have been reported to be affected with aging and disease. To our knowledge this is the first study showing prevention of cognitive decline following administration of either rosemary extract with carnosic acid or spearmint extract with rosmarinic acid in a mouse model of cognitive decline due to accelerated aging. These findings suggest that rosemary extract with carnosic and spearmint extract with rosmarinic acid are potential treatments for age-associated cognitive decline.

All three extracts improved learning and memory in at least two of the three tasks. Rosemary extract (with CA) had an effect on both declarative and procedural memory. The T-maze and object recognition both tested declarative memory. The operant lever press tests procedural learning and memory. Spearmint extract (with RA) improved all declarative memory tasks tested (T-maze acquisition and learning and object recognition) but had no effect on procedural operant learning and memory. None of the compounds had an effect on body weight. The use of CA and/or RA is now directly linked to outcome measures of cognition. There are rosemary supplements that discuss energy and cognition, but none are supported by scientific studies and none directly link the cause to carnosic acid. This is shown in our study as increasing the carnosic acid content increased the efficacy of the extract.

Cognitive behavioral outcome measures related to learning or memory have not been linked to CA, in addition learning outcome measures have not been linked to RA.

These results are surprising due to the nature of how two totally different molecules, with different chemical structures and polarities are able to cross the blood brain barrier and penetrate different regions of the brain. Once these compounds penetrate various regions of the brain they result in significant performance changes in cognitive performance. Prior to this research, it was thought that these molecules only had general non-specific antioxidant effects in the brain. Previously these molecules have not been associated with specific cognitive functional effects resulting from different regions of the brain. For the first time, this research demonstrates that they can affect behavioral cognitive performance outcomes. In addition, the two compounds affect different performance outcomes differently demonstrating a very specific interaction with certain brain regions responsible for those cognitive outcomes. These results demonstrate specificity of these molecules to act independently of the general non-specific antioxidant effects attributed to other molecules.

### COMPARATIVE EXAMPLE 2

### 90-day Toxicity Study of Carnosic Acid

The purpose of this study was to evaluate the toxicity of the test article, a suspension (in sunflower oil) of rosemary extract containing approximately 10% carnosic acid (the active ingredient), when administered once or twice daily via oral gavage to rats for at least 90 days.

Male and female Hsd:Sprague Dawley SD rats were assigned to groups, and doses were administered as indicated in the following table. Animals were dosed via oral gavage.

| | No. of Animals^{a} | | CA Dose Level^{a} | | CA Dose Concentration^{b} |
|---|---|---|---|---|---|
| Group | Male | Female | (mg/kg/dose) | (mg/kg/day) | (mg/mL) |
| 1 (Control, BID dosing)^{c} | 10 | 10 | 0 | 0 | 0 |
| 2 (Low, BID dosing) | 10 | 10 | 32.5 | 65 | 6.5 |
| 3 (Mid, BID dosing) | 10 | 10 | 65 | 130 | 13.0 |
| 4 (Mid, SID dosing) | 10 | 10 | 130 | 130 | 13.0 |
| 5 (High, BID dosing) | 10 | 10 | 90 | 180 | 18.0 |

| | | | | | |
|---|---|---|---|---|---|
| CA = Carnosic acid; the active ingredient in the test article. BID = Twice daily; doses on each day were administered approximately 6 hours apart (based on last animal dosed/sex/group). SID = Once daily. a Animals were dosed at 10 mL/kg/day; doses for BID dosed animals were administered at 5 mL/kg/dose. b Concentrations were corrected for lot specific CA content. c Group 1 received vehicle control article (sunflower oil) only. | | | | | |

Assessment of toxicity was based on mortality, clinical signs, body weight, body weight change, food consumption, ophthalmic examinations, functional observation battery (FOB), and clinical and anatomic pathology.

Oral administration of rosemary extract containing approximately 10% carnosic acid in sunflower oil to Sprague Dawley rats was well tolerated at dose levels of <180 mg/kg/day. The frequency of dosing (SID or BID) in animals given 130 mg/kg/day did not appear to have any substantial difference on the nature or magnitude of effects. Doses >65 mg/kg/day resulted in nonadverse test article-related effects on hematology and clinical chemistry parameters; organ weight changes; and microscopic findings in liver, kidney, nonglandular stomach, colon, and cecum. Based on this pattern of findings, 180 mg carnosic acid/kg/day is considered the no observed adverse effect level (NOAEL) when given orally for 90 consecutive days. With a NOAEL of 180 mg/kg/day of carnosic acid and a CA concentration within the extract of exactly 11.55%, the NOAEL would translate to 1,558 mg/kd/day of the rosemary extract. Using a 100-fold safety factor, this would translate to 15.58 mg/kg/day human equivalent dose, or 1091 mg for a 70 kg human.

### EXAMPLE 3

### 90-day Toxicity Study of Rosmarinic Acid

The purpose of this study was to evaluate the toxicity of the test article, a dry extract of rosemary containing approximately 15% (w/w) rosmarinic acid (the active ingredient) dissolved in distilled water, when administered once daily via oral gavage to rats for at least 90 days.

Male and female Hsd:Sprague Dawley SD rats were assigned to groups, and doses were administered as indicated in the following table. Animals were dosed via oral gavage.

| | No. of Animals^{a} | | RA Dose Level^{a} | RA Dose Concentration |
|---|---|---|---|---|
| Group | Male | Female | (mg/kg/day) | (mg/mL) |
| 1 (Control)b | 10 | 10 | 0 | 0 |
| 2 (Low) | 10 | 10 | 65 | 6.5 |
| 3 (Mid) | 10 | 10 | 130 | 13 |
| 5 (High) | 10 | 10 | 300 | 30 |

| | | | | |
|---|---|---|---|---|
| RA = Rosmarinic acid; the active ingredient in the test article. a Animals were dosed at 10 mL/kg/day. b Group 1 received vehicle control article (distilled water) only. | | | | |

Assessment of toxicity was based on mortality, clinical signs, body weight, body weight change, food consumption, ophthalmic examinations, functional observation battery (FOB), and clinical and anatomic pathology.
Daily oral administration of the Dry Spearmint Extract (containing approximately 15% RA as active ingredient) to male and female Sprague Dawley rats at doses up to 300 mg RA/kg b.wt/day for 90 days was well tolerated. No treatment related clinical signs or adverse effects were observed in any of the parameters studied including body weight, food consumption, neurological parameters, hematology, clinical chemistry, gross and histopathology. Increased weights were observed in the pituitary and thyroid lands, however there were within the historical norms for the age and strain and had no corresponding histopathology changes.Hence, the "No Observed Adverse Effect Level (NOAEL)" for the test item under the testing conditions and doses employed was found to be 300 mg RA/kg b.wt/day (corresponding to 1948.2 mg/kg b.wt/day of the Dry Spearmint Extract). Using a 100 fold safety factor, this would correspond to a NOAEL of 19.48 mg/kg/day of dry spearmint extract for the human equivalent dose, which for a 70 kg human would be equivalent to 1363.74 mg/day.

The foregoing description and drawings merely explain and illustrate the invention.

## Claims

1. A water extract of a spearmint plant comprising 5% rosmarinic acid for use by oral administration in an amount of said extract between 0.01 and 50 mg/kg/day in therapeutically enhancing, improving or sustaining cognitive health and/or cognitive function in mammals experiencing normal aging cognitive changes.

2. A water extract of a spearmint plant for use according to claim 1, wherein said mammals are selected from the list consisting of humans and companion animals.

3. A water extract of a spearmint plant for use according to claim 1, wherein cognitive health and/or cognitive function for rosmarinic acid refers to declarative learning through improvement of focus, attention, and alertness.

4. A water extract of a spearmint plant for use according to claim 1, wherein cognitive health and/or cognitive function for rosmarinic acid refers to declarative memory, both short-term and long-term memory.

5. A water extract of a spearmint plant for use according to claim 1, wherein cognitive health and /or cognitive function refers to executive function.

6. A water extract of a spearmint plant comprising 5% rosmarinic acid for use by oral administration in an amount of said extract of between 0.01 and 50 mg/kg/day in the therapeutic maintenance of normal cognitive function under disruption of circadian rhythms as occurs in a condition selected from the list consisting of time zone changes, pregnancy, medications, changes in routine and shift work.

7. A water extract of a spearmint plant comprising 5% rosmarinic acid for use by oral administration in an amount of said extract of between 0.01 and 50 mg/kg/day in therapeutically treating or preventing a decline in cognitive health and/or cognitive function in a mammal experiencing normal aging cognitive changes.

## Patentansprüche

1. Wasserextrakt einer Pflanze der Grünen Minze, umfassend 5 % Rosmarinsäure, zur Verwendung durch orale Verabreichung in einer Menge des Extrakts zwischen 0,01 und 50 mg/kg/Tag bei der therapeutischen Stärkung, Verbesserung oder Erhaltung der kognitiven Gesundheit und/oder der kognitiven Funktion bei Säugetieren, die normale altersbedingte kognitive Veränderungen erfahren.

2. Wasserextrakt einer Pflanze der Grünen Minze zur Verwendung nach Anspruch 1, wobei die Säugetiere aus der Liste, die aus Menschen und Heimtieren besteht, ausgewählt sind.

3. Wasserextrakt einer Pflanze der Grünen Minze zur Verwendung nach Anspruch 1, wobei sich die kognitive Gesundheit und/oder die kognitive Funktion für Rosmarinsäure auf das deklarative Lernen durch Verbesserung von Fokus, Aufmerksamkeit und Wachheit bezieht.

4. Wasserextrakt einer Pflanze der Grünen Minze zur Verwendung nach Anspruch 1, wobei sich die kognitive Gesundheit und/oder die kognitive Funktion für Rosmarinsäure auf das deklarative Gedächtnis, sowohl das Kurzzeit- als auch das Langzeitgedächtnis bezieht.

5. Wasserextrakt einer Pflanze der Grünen Minze zur Verwendung nach Anspruch 1, wobei sich die kognitive Gesundheit und/oder die kognitive Funktion auf exekutive Funktionen bezieht.

6. Wasserextrakt einer Pflanze der Grünen Minze, umfassend 5 % Rosmarinsäure, zur Verwendung durch orale Verabreichung in einer Menge des Extrakts zwischen 0,01 und 50 mg/kg/Tag bei der therapeutischen Erhaltung der normalen kognitiven Funktion unter einer Störung der zirkadianen Rhythmen, wie sie in einem Zustand auftritt, der aus der Liste, die aus Wechseln der Zeitzone, Schwangerschaft, Medikationen, Veränderungen des Tagesablaufs und Schichtarbeit besteht, ausgewählt ist.

7. Wasserextrakt einer Pflanze der Grünen Minze, umfassend 5 % Rosmarinsäure, zur Verwendung durch orale Verabreichung in einer Menge des Extrakts zwischen 0,01 und 50 mg/kg/Tag bei der therapeutischen Behandlung oder Vorbeugung einer Verschlechterung der kognitiven Gesundheit und/oder der kognitiven Funktion bei einem Säugetier, das normale altersbedingte kognitive Veränderungen erfährt.

## Revendications

1. Extrait aqueux d'une plante de menthe verte comprenant 5 % d'acide rosmarinique destiné être utilisé par administration par voie orale dans une quantité dudit extrait entre 0,01 et 50 mg/kg/jour pour favoriser, améliorer ou entretenir thérapeutiquement la santé cognitive et/ou la fonction cognitive chez des mammifères faisant l'expérience de changements cognitifs normaux liés au vieillissement.

2. Extrait aqueux d'une plante de menthe verte destiné à être utilisé selon la revendication 1, dans lequel lesdits mammifères sont sélectionnés parmi la liste constituée par des humains et des animaux de compagnie.

3. Extrait aqueux d'une plante de menthe verte destiné à être utilisé selon la revendication 1, dans lequel la santé cognitive et/ou la fonction cognitive pour l'acide rosmarinique fait référence à la mémoire déclarative par l'amélioration de la concentration, de l'attention, et de la vivacité.

4. Extrait aqueux d'une plante de menthe verte destiné à être utilisé selon la revendication 1, dans lequel la santé cognitive et/ou la fonction cognitive pour l'acide rosmarinique fait référence à la mémoire déclarative, la mémoire à la fois à court terme et à long terme.

5. Extrait aqueux d'une plante de menthe verte destiné à être utilisé selon la revendication 1, dans lequel la santé cognitive et/ou la fonction cognitive fait référence à la fonction exécutive.

6. Extrait aqueux d'une plante de menthe verte comprenant 5 % d'acide rosmarinique destiné être utilisé par administration par voie orale dans une quantité dudit extrait entre 0,01 et 50 mg/kg/jour dans le maintien thérapeutique de la fonction cognitive normale en cas de perturbation des rythmes circadiens telle qu'elle apparaît dans une affection sélectionnée parmi la liste constituée par les décalages horaires, la grossesse, les médicaments, les changements de routine et le travail posté.

7. Extrait aqueux d'une plante de menthe verte comprenant 5 % d'acide rosmarinique destiné être utilisé par administration par voie orale dans une quantité dudit extrait entre 0,01 et 50 mg/kg/jour dans le traitement ou la prévention thérapeutique d'une dégradation de la santé cognitive et/ou de la fonction cognitive chez un mammifère faisant l'expérience de changements cognitifs normaux liés au vieillissement.
